# FASCICULE DE BREVET EUROPEEN

(11) **EP 0 384 843 B1**
(45) Date de publication et mention de la délivrance du brevet: **31.08.1994**
(21) Numéro de dépôt: 90400494.2
(22) Date de dépôt: 22.02.1990
(51) Int. Cl.: C07D 211/96, A61K 31/44, C07C 311/32, C07D 295/088

(54) **Dérivés de la 1-arylsulfonyl 2-pipéridinone, leur procédé et les intermédiaires de préparation, leur application comme médicaments et les compositions les renfermant**
1-Arylsulfonyl-2-piperidinon-Derivate, Verfahren und Zwischenprodukte zu ihrer Herstellung, ihre Verwendung als Arzneimittel und sie enthaltende Zusammensetzungen
1-Arylsulfonyl-2-piperidinone derivatives, process and intermediates for their preparation, their pharmaceutical use and compositions containing them

(30) Priorité: 22.02.1989 IT 1952489
(43) Date de publication de la demande: 29.08.1990
(73) Titulaire: ROUSSEL-UCLAF, 75007 Paris (FR)
(72) Inventeur: Toja, Emilio, Milano (IT); Barzaghi, Fernando, I-20052 Monza (Milan) (IT); Galliani, Giulio, 1 Monza (IT)
(74) Mandataire: Tonnellier, Marie-José

(56) Documents cités:
- EP-A- 0 068 968
- EP-A- 0 138 721
- EP-A- 0 335 758
- US-A- 2 142 847
- BULLETIN OF THE CHEMICAL SOCIETY OF JAPAN, vol. 42, no. 7, juillet 1969, pages 1955-1958, Tokio, JP; T. SATO et al.: "Reactions of cycloalkanone oxime p-toluenesulfonates"
- TETRAHEDRON, vol. 44, no. 2, janvier 1988, pages 613-618, Pergamon Journals Ltd, Oxford, GB; D. TANNER et al.: "A mild and efficient method for the preparation of n-tosyl amides and lactams"

## Description

La présente invention concerne de nouveaux dérivés de la 1-arylsulfonyl 2-pipéridinone, leur procédé et les intermédiaires de préparation, leur application comme médicaments et les compositions les renfermant.

On connaissait les benzene sulfonyl lactames présentant des propriétés pharmacologiques diverses, voir à ce sujet E.A.O. 138,721, Bulletin of The Chemical Society of Japan, vol. 42 p. 1955-8 (1969), Tetrahedron vol. 44 p. 613-8 (1988).

Le brevet européen 0 335 758 décrit des dérivés de la 1-aryl sulfonyl 2-pyrrolidone présentant des propriétés anti-muscariniques.

L'invention a pour objet les composés de formule (I) : 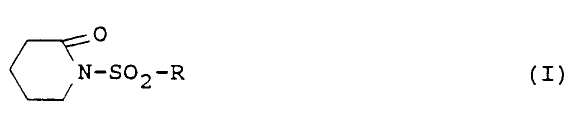
dans laquelle R représente
un radical 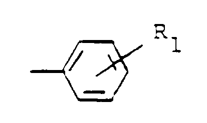
dans laquelle R₁ en position quelconque sur le noyau phényle représente un radical 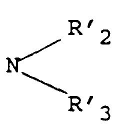
dans lequel R'₂ et R'₃ identiques ou différents représentent un radical alcoyle linéaire ou ramifié, renfermant jusqu'à 8 atomes de carbone, ou forment ensemble avec l'atome d'azote auquel ils sont liés un radical hétérocyclique choisi parmi les radicaux pipéridyle, pipérazinyle, morpholinyle, pyrrolidinyle, hexahydroazépinyle et octahydroazocinyle.

Par radical alcoyle, on entend de préférence un radical alcoyle renfermant de 1 à 6 atomes de carbone, par exemple le radical méthyle, éthyle, n-propyle, isopropyle, n-butyle, isobutyle, tert-butyle, n-pentyle, n-hexyle, cyclopropyle, cyclobutyle, cyclopentyle ou cyclohexyle.

Par radical alcoyle insaturé, on entend de préférence un radical éthényle, propényle ou butényle.

Parmi les composés préférés de l'invention, on peut citer les composés dans lesquels le radical R₁ est en position 4.

Parmi les composés préférés de l'invention, on peut citer tout particulièrement les composés dans lesquels R'₂ et R'₃, identiques ou différents, représentent un radical alcoyle renfermant jusqu'à 4 atomes de carbone, ainsi que ceux dans lesquels R₁ représente un radical pipéridinyle ou hexahydroazépine.

L'invention a plus particulièrement pour objet les composés dont la préparation est donnée ci-après dans la partie expérimentale et tout particulièrement le composé de l'exemple 2 ou 4.

Les composés de formule (I) présentent d'intéressantes propriétés pharmacologiques et notamment une activité antimuscarinique spécifique et sélective.

L'invention a donc pour objet les produits de formule (I) en tant que médicaments, utiles notamment pour le traitement des troubles spasmodiques divers en gastro-entérologie, en gynécologie, en obstétrique, en urologie, en hépatologie et en radiologie.

L'invention a plus particulièrement pour objet en tant que médicament, les composés préférés cités ci-dessus et tout spécialement le produit de l'exemple 2, 4 ou 5.

La posologie usuelle est variable selon l'affection en cause, le sujet traité et la voie d'administration, elle peut être comprise entre 10 mg et 1 g par jour, par exemple entre 30 et 60 mg par jour en une ou plusieurs prises pour le produit de l'exemple 2 administré par voie orale.

La présente invention a également pour objet les compositions pharmaceutiques renfermant comme principe actif au moins un produit de formule (I). Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I), caractérisé en ce que l'on soumet un composé de formule (II) :

R-SO₂-Hal (II)

dans laquelle Hal représente un atome de chlore ou de brome et R conserve la signification indiquée précédemment, à l'action de l'acide 5-amino valérique (III) : 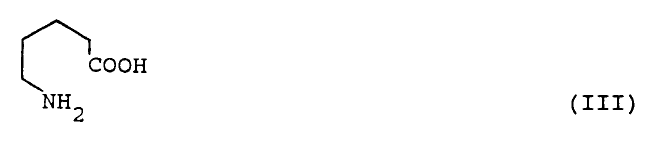
pour obtenir un composé de formule (IV) : 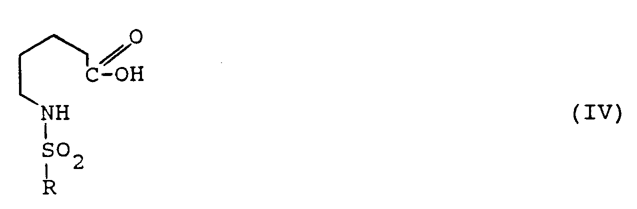
que l'on cyclise pour obtenir le produit de formule (I) correspondant.

Dans un mode de réalisation préféré du procédé de l'invention :
- La condensation du composé (II) avec l'acide 5-amino valérique est réalisée dans les conditions classiques de SCHOTTEN-BAUMANN, en présence d'une base comme la soude ou la potasse par exemple au sein d'un solvant organique comme le tétrahydrofuranne.
- La cyclisation du composé de formule (IV) est réalisée au moyen d'un anhydride d'acide, comme l'anhydride acétique ou encore au moyen d'autres agents de condensation comme l'acide sulfurique, l'anhydride phosphorique, l'acide phosphorique, l'acide métaphosphorique, le dicyclohexylcarbodiimide en présence de pyridine ou la bis-(triméthylsilyl) amine avec le chlorure de triméthylsilyle.

Les composés de formule (II) utilisés comme produits de départ sont connus de façon générale [cf. Houben Weyl, 4ème éd., vol. 9, Ch 18 (1955)].

La préparation des produits utilisés comme produits de départ des exemples 3, 4, 5 et 6 est indiquée ci-après.

Les exemples suivants illustrent l'invention.

### Exemple 1 :1-[4-(diméthylamino) benzènesulfonyl] 2-pipéridinone.

### Stade A : Acide 5-(4-diméthylaminobenzènesulfonylamino) valérique.

Dans une solution comprenant 2,34 g d'acide 5-amino valérique et 2,4 g de soude en solution dans 24 cm3 d'eau, on ajoute 4,4 g de chlorure de (4-diméthylamino benzène sulfonyle (Chem. Ber. 43, 3038, (1910)) puis 24 cm3 de tétrahydrofuranne afin d'obtenir une solution. La température s'élève à 36°C. On maintient 4 heures sous agitation, évapore le tétrahydrofuranne, acidifie le milieu réactionnel à l'aide d'acide acétique, extrait au chloroforme, sèche la phase organique et élimine les solvants sous pression réduite. On obtient 3,7 g de produit attendu. F = 105-110°C. Après cristallisation dans le mélange isopropanol-eau (1-1), on obtient le produit fondant à 118-120°C.

| Analyse : C₁₃H₂₀N₂O₄S | | | | | | |
|---|---|---|---|---|---|---|
| Calculé | C% | 51,98 | H% | 6,71 | N% | 9,33 |
| Trouvé | | 51,85 | | 6,77 | | 9,28 |

### Stade B : 1-[4-(diméthylamino) benzènesulfonyl] 2-pipéridinone.

On chauffe 3 heures au reflux 4,8 g de produit obtenu au stade A avec 4,8 g d'acétate de sodium dans 48 cm³ d'anhydride acétique. On refroidit à température ambiante, évapore à sec, reprend le résidu dans 50 cm3 d'eau, filtre, sèche et obtient 3,7 g de produit brut attendu. F = 165-170°C. Après cristallisation dans l'éthanol, on obtient 2,9 g de produit pur.
F = 169-171°C.

| Analyse : C₁₃H₁₈N₂O₃S | | | | | | |
|---|---|---|---|---|---|---|
| Calculé | C% | 55,30 | H% | 6,43 | N% | 9,92 |
| Trouvé | | 55,5 | | 6,5 | | 9,8 |

### Exemple 2 : 1-[4-(diéthylamino) benzènesulfonyl] 2-pipéridinone.

### Stade A : Acide 5-(4-diéthylaminobenzènesulfonylamino) valérique.

Dans une solution comprenant 1,17 g d'acide 5-amino valérique et 1,2 g de soude en solution dans 12 cm³ d'eau, on ajoute 2,5 g de chlorure de 4-diéthylamino benzène sulfonyle puis 12 cm³ de tétrahydrofuranne afin d'obtenir une solution. On maintient 2 heures sous agitation à température ambiante, évapore le tétrahydrofuranne, acidifie le milieu réactionnel à l'aide d'acide acétique, extrait au chloroforme, sèche la phase organique et élimine les solvants sous pression réduite. On obtient 2,2 g de produit attendu. F = 106-108°C. Après cristallisation dans le mélange isopropanol-eau (1-1), on obtient le produit fondant à 109-110°C.

| Analyse : C₁₅H₂₄N₂O₄S | | | | | | |
|---|---|---|---|---|---|---|
| Calculé | C% | 54,86 | H% | 7,36 | N% | 8,53 |
| Trouvé | | 54,63 | | 7,28 | | 8,65 |

### Stade B : 1-[4-(diéthylamino) benzènesulfonyl] 2-pipéridinone.

On chauffe 2 heures au reflux 2 g de produit obtenu au stade A avec 2 g d'acétate de sodium dans 20 cm³ d'anhydride acétique. On refroidit à température ambiante, évapore à sec, reprend le résidu dans un mélange de chloroforme et d'eau, sépare la phase organique, la sèche et obtient après élimination des solvants 1,8 g de produit brut attendu. F = 118-120°C. Après cristallisation dans l'isopropanol, on obtient 1,2 g de produit pur. F = 122-123°C.

| Analyse : C₁₅H₂₂N₂O₃S | | | | | | |
|---|---|---|---|---|---|---|
| Calculé | C% | 58,04 | H% | 7,14 | N% | 9,02 |
| Trouvé | | 58,28 | | 7,34 | | 9,09 |

Le chlorure de 4-(diéthylamino) benzènesulfonyle utilisé comme produit de départ a été préparé comme indiqué dans la demande de brevet européen publié sous le n° 0 033 578.

### Exemple 3 : 1-[4-(dipropylamino) benzènesulfonyl] 2-pipéridinone.

### Stade A : Acide 5-(4-dipropylaminobenzènesulfonylamino) valérique.

Dans une solution comprenant 2,76 g d'acide 5-amino valérique et 2,82 g de soude en solution dans 28 cm³ d'eau, on ajoute 6,5 g de chlorure de 4-dipropylamino benzène sulfonyle en solution puis 28 cm³ de tétrahydrofuranne. On maintient 2 heures sous agitation, évapore le tétrahydrofuranne, acidifie le milieu réactionnel à l'aide d'acide acétique, filtre le solide formé, le lave à l'eau et le sèche. On obtient 6 g de produit attendu. F = 95-98°C. Après cristallisation dans le mélange isopropanol-eau (1-1), on obtient 5 g de produit fondant à 98-99°C.

| Analyse : C₁₇H₂₈N₂O₄S | | | | | | |
|---|---|---|---|---|---|---|
| Calculé | C% | 57,28 | H% | 7,92 | N% | 7,86 |
| Trouvé | | 57,34 | | 8,0 | | 7,91 |

### Stade B : 1-[4-(dipropylamino) benzènesulfonyl] 2-pipéridinone.

On chauffe 2 heures au reflux 5 g de produit obtenu au stade A avec 5 g d'acétate de sodium dans 50 cm³ d'anhydride acétique. On refroidit à température ambiante, évapore à sec, reprend le résidu dans 50 cm³ d'eau, sèche et obtient 4,4 g de produit attendu. F = 105-108°C après chromatographie sur silice (éluant : acétate d'éthyle-n-hexane 1-1). Après cristallisation dans l'isopropanol, on obtient 3,2 g de produit pur. F = 110-111°C.

| Analyse : C₁₇H₂₆N₂O₃S | | | | | | |
|---|---|---|---|---|---|---|
| Calculé | C% | 60,32 | H% | 7,74 | N% | 8,28 |
| Trouvé | | 60,48 | | 7,81 | | 8,36 |

Le chlorure de 4-dipropylaminobenzènesulfonyle a été préparé comme suit :
On ajoute, à une température comprise entre +5° et +10°C, 10,2 g de N,N-dipropylaniline (Annalen der Chemie 214, 168) dans une solution comprenant 10,86 g (soit 8,86 cm³) de chlorosulfonate de triméthylsilyle et 50 cm³ de dichlorométhane. On laisse revenir à température ambiante, évapore à sec, reprend le résidu dans l'acétone, filtre le produit solide et le sèche. On obtient 4,9 g d'acide que l'on reprend dans 100 cm³ de dichlorométhane et ajoute 2,63 g de pentachlorure de phosphore et chauffe au reflux pendant 4 heures. On refroidit à température ambiante, évapore à sec, reprend l'huile résiduelle dans du benzène et de l'eau. On sépare la phase organique, la sèche et évapore les solvants. On obtient 4,5 g d'huile que l'on utilise telle quelle pour le stade suivant.

### Exemple 4 : 1-[4-(1-pipéridinyl) benzènesulfonyl] 2- pipéridinone.

### Stade A : Acide 5-(4-pipéridinylbenzènesulfonylamino) valérique.

Dans une solution comprenant 3,51 g d'acide 5-amino valérique et 3,6 g de soude en solution dans 35 cm³ d'eau, on ajoute 7,8 g de chlorure de 4-pipéridinyl benzène sulfonyle puis 35 cm³ de tétrahydrofuranne afin d'obtenir une solution. La température s'élève à 35°C. On maintient 4 heures sous agitation à température ambiante, évapore le tétrahydrofuranne, acidifie le milieu réactionnel à l'aide d'acide acétique, dilue avec 100 cm³ d'eau, extrait au chloroforme, sèche la phase organique et élimine les solvants sous pression réduite. On obtient 5,8 g de produit attendu. F = 115-120°C. Après cristallisation dans le mélange isopropanol-eau (1-1), on obtient le produit fondant à 120-122°C.

| Analyse : C₁₆H₂₄N₂O₄S | | | | | | |
|---|---|---|---|---|---|---|
| Calculé | C% | 56,45 | H% | 7,10 | N% | 8,23 |
| Trouvé | | 56,19 | | 7,05 | | 8,06 |

### Stade B : 1-[4-(1-pipéridinyl) benzènesulfonyl] 2-pipéridinone.

On chauffe 4 heures au reflux 5 g de produit obtenu au stade A avec 5 g d'acétate de sodium dans 50 cm³ d'anhydride acétique. On refroidit à température ambiante, évapore à sec, reprend le résidu dans 50 cm3 d'eau, filtre, sèche et obtient 4,4 g de produit brut attendu. F = 140-145°C. Après cristallisation dans l'éthanol, on obtient 3,4 g de produit pur. F = 145-146°C.

| Analyse : C₁₆H₂₂N₂O₃S | | | | | | |
|---|---|---|---|---|---|---|
| Calculé | C% | 59,60 | H% | 6,88 | N% | 8,69 |
| Trouvé | | 59,51 | | 6,97 | | 8,63 |

Le chlorure de 4-pipéridinylaminobenzène sulfonyle a été préparé comme suit :
A une solution comprenant 8,46 g d'anhydride sulfurique dans 45 cm³ de chlorure de méthylène refroidie entre 0°C et - 5°C, on ajoute goutte à goutte 9,3 g de dioxane puis 17,1 g de N-phényl pipéridine dissous dans 45 cm³ de chlorure de méthylène. On laisse revenir à température ambiante, puis chauffe au reflux pendant 1 heure, évapore à sec, neutralise avec une solution de carbonate de sodium à 10%, concentre la phase aqueuse et sèche le résidu que l'on traite par 200 cm³ de chlorure de phosphoryle et 21,8 g de pentachlorure de phosphore pendant 12 heures à température ambiante. On évapore à sec, reprend le résidu au chloroforme avec un peu d'eau, sépare la phase organique, la sèche sur sulfate de sodium, filtre et évapore le solvant. On obtient 19 g de produit attendu que l'on utilise tel quel au stade suivant.

### Exemple 5 : 1-[4-(1-hexahydroazépinyl) benzènesulfonyl] 2-pipéridinone.

### Stade A : Acide 5-(4-1-hexahydroazépinyl benzènesulfonylamino) valérique.

Dans une solution comprenant 2,56 g d'acide 5-amino valérique et 2,63 g de soude en solution dans 60 cm³ d'eau, on ajoute 6 g de chlorure de 4-hexahydroazépine benzène sulfonyle puis 60 cm³ de tétrahydrofuranne afin d'obtenir une solution. On maintient 2 heures sous agitation à température ambiante, évapore le tétrahydrofuranne, acidifie le milieu réactionnel à l'aide d'acide acétique, filtre le précipité, le lave à l'eau, le sèche et obtient 4,65 g de produit attendu. F = 135-140°C. Après cristallisation dans l'isopropanol, on obtient le produit fondant à 139-140°C.

| Analyse : C₁₇H₂₆N₂O₄S | | | | | | |
|---|---|---|---|---|---|---|
| Calculé | C% | 57,60 | H% | 7,39 | N% | 7,90 |
| Trouvé | | 57,46 | | 7,37 | | 7,98 |

### Stade B : 1-[4-(1-hexahydroazépinyl) benzènesulfonyl] 2-pipéridinone.

On chauffe 1 heure au reflux 4 g de produit obtenu au stade A avec 4 g d'acétate de sodium dans 80 cm³ d'anhydride acétique. On refroidit à température ambiante, évapore à sec, reprend le résidu dans 60 cm³ d'eau, filtre, sèche et obtient 3,5 g de produit attendu. Après cristallisation dans l'isopropanol, on obtient 2,3 g de produit. F = 164-165°C.

| Analyse : C₁₇H₂₄N₂O₃S | | | | | | |
|---|---|---|---|---|---|---|
| Calculé | C% | 60,69 | H% | 7,19 | N% | 8,33 |
| Trouvé | | 60,75 | | 7,09 | | 8,41 |

Le chlorure de 4-hexahydroazépinyl benzène sulfonyle a été préparé comme suit :
A une solution comprenant 2,64 g d'anhydride sulfurique dans 78 cm³ de chlorure de méthylène refroidie entre +5°C et +10°C, on ajoute goutte à goutte 2,91 g de dioxane puis 5,26 g de 1-phényl hexahydroazépine (Tetrahedron 41 (1985) p. 101-106 dans 53 cm³ de chlorure de méthylène. On laisse revenir à température ambiante, puis chauffe au reflux pendant 2 heures, refroidit de nouveau à température ambiante, ajoute 200 cm³ d'éther éthylique à la suspension, filtre le précipité, le lave à l'éther, le sèche et obtient 7,2 g d'acide (F = 235°C décomp) que l'on traite par 36 cm³ de chlorure de phosphoryle dans 36 cm³ de chlorure de méthylène et par 5,87 g de pentachlorure de phosphore pendant 4 heures à température ambiante. On évapore à sec, reprend le résidu avec 100 cm³ d'eau et 150 cm³ de chloroforme, sépare la phase organique, la sèche sur sulfate de sodium, filtre et évapore le solvant. On obtient 6,6 g de produit attendu. F = 85-88°C.

### Exemple 6 : 1-[4-(1-azacyclooctyl) benzènesulfonyl] 2-pipéridinone.

### Stade A : Acide 5-(4-(1-azacyclooctyl) benzènesulfonylamino) valérique.

Dans une solution comprenant 1,82 g d'acide 5-amino valérique et 1,87 g de soude en solution dans 45 cm³ d'eau, on ajoute 4,5 g de chlorure de 4-azacyclooctyl benzène sulfonyle puis 45 cm³ de tétrahydrofuranne afin d'obtenir une solution. On maintient 2 heures sous agitation à température ambiante, évapore le tétrahydrofuranne, acidifie le milieu réactionnel à l'aide d'acide acétique, filtre le précipité, le lave à l'eau, le sèche et obtient 3,6 g de produit attendu. F = 149-150°C. Après cristallisation dans le mélange isopropanol-eau (1-1), on obtient le produit fondant à 153-154°C.

| Analyse : C₁₈H₂₈N₂O₄S | | | | | | |
|---|---|---|---|---|---|---|
| Calculé | C% | 58,67 | H% | 7,66 | N% | 7,60 |
| Trouvé | | 58,76 | | 7,56 | | 7,74 |

### Stade B : 1-[4-(1-azacyclooctyl) benzènesulfonyl] 2-pipéridinone.

On chauffe 1 heure au reflux 3,4 g de produit obtenu au stade A avec 3,4 g d'acétate de sodium dans 68 cm³ d'anhydride acétique. On refroidit à température ambiante, évapore à sec, reprend le résidu dans 50 cm³ d'eau, filtre, sèche et obtient 2,87 g de produit attendu. F = 150-152°C. Après cristallisation dans l'isopropanol, on obtient 1,9 g de produit pur. F = 152-153°C.

| Analyse : C₁₈H₂₆N₂O₃S | | | | | | |
|---|---|---|---|---|---|---|
| Calculé | C% | 61,68 | H% | 7,48 | N% | 7,99 |
| Trouvé | | 61,50 | | 7,50 | | 7,86 |

Le chlorure de 4-azacyclooctyl benzène sulfonyle a été préparé comme suit :

### STADE A : 1-azacyclooctyl benzène.

On chauffe à 100°C pendant 20 minutes 4,3 g d'amide de sodium à 50% dans le toluène en suspension dans 18,9 cm³ (soit 16,9 g) d'heptaméthylènamine puis ajoute goutte à goutte 7,85 g (soit 5,03 cm³) de bromobenzène. On chauffe le milieu réactionnel au reflux pendant 18 heures, refroidit à température ambiante, ajoute 50 cm³ d'eau. On ajoute 100 cm³ de benzène, sépare la phase organique, extrait à l'aide d'une solution aqueuse d'acide chlorhydrique à 5%, alcalinise à l'aide d'une solution aqueuse de soude à 20%, sépare la phase huileuse, l'extrait à l'éther éthylique, la sèche et évapore le solvant. Après distillation du résidu (Eb. 118-120°C sous 0,5 mm de Hg), on obtient 9,4 g de produit attendu.

| Analyse : C₁₃H₁₉N | | | | | | |
|---|---|---|---|---|---|---|
| Calculé | C% | 82,48 | H% | 10,12 | N% | 7,40 |
| Trouvé | | 82,28 | | 9,95 | | 7,52 |

### STADE B : Chlorure de 4-azacyclooctyl benzène sulfonyle.

A une solution comprenant 3,97 g d'anhydride sulfurique dans 40 cm³ de chlorure de méthylène et refroidie entre +5°C et +10°C, on ajoute goutte à goutte 4,36 g de dioxane puis 9,4 g de 1-azacyclooctyl benzène. On laisse revenir à température ambiante, puis chauffe au reflux pendant 1 heure. On refroidit de nouveau à température ambiante, dilue avec 200 cm³ d'éther éthylique, filtre et obtient après séchage 13,3 g d'acide que l'on traite avec 10,34 g de pentachlorure de phosphore dans 50 cm³ de chlorure de phosphoryle et 50 cm³ de chlorure de méthylène pendant 3 heures à température ambiante. On évapore à sec, reprend le résidu avec de l'eau et du chloroforme, sépare la phase organique, la sèche sur sulfate de sodium, filtre et évapore. On obtient 13 g de produit attendu.

### Exemples de compositions pharmaceutiques.

a) On a préparé des comprimés répondant à la formule suivante :

| | |
|---|---|
| - Produit de l'exemple 2 | 10 mg |
| - Excipient q.s. pour un comprimé terminé a | 300 mg |
| (Détail de l'excipient : lactose, amidon de blé, amidon traité, amidon de riz, stéarate de magnésium, talc). | |

b) On a préparé des gélules répondant à la formule suivante :

| | |
|---|---|
| - Produit de l'exemple 4 | 20 mg |
| - Excipient q.s. pour une gélule terminée à | 300 mg |
| (Détail de l'excipient : talc, stéarate de magnésium, aérosil). | |

### ETUDES BIOCHIMIQUES ET PHARMACOLOGIQUES

### 1) Liaison à des différents récepteurs cérébraux.

### a) Récepteur muscarinique 1.

Sa préparation est faite à partir de Cortex prélevés sur des cerveaux de rats mâles pesant 150 à 200 g, (Iffa Crédo) broyé au Polytron dans un tampon Na/K 10 mM pH 7,4. Après incubation (aliquotes de 0,5 ml d'homogénat) pendant 60 minutes à 25°C en présence de 0,25 nM de ³H pirenzépine soit seule, soit avec le produit à tester, soit avec un excès de pirenzépine à 10⁻⁵M (pour déterminer la radioactivité fixée non spécifique), les incubats sont refroidis et filtrés.

La filtration est effectuée sur filtres Whatman GF/C prélavés dans une solution de polyéthylène imine à 0,05%. Les filtres sont rincés par 3 x 5 ml de tampon phosphate Na/K 10 mM pH 7,4 puis on effectue les mesures par scintillation liquide.

### b) Récepteur muscarinique 2.

La préparation est effectuée à partir de cerveaux de rats mâles pesant 150 à 200 g (Iffa Crédo).

Les cerveaux sont broyés (Téflon-verre) dans une solution de sucrose 0,32 M. L'homogénat est centrifugé 10 minutes à 1000 g (0 - 4°C).

Le surnageant obtenu est recueilli et centrifugé à 30 000 g pendant 15 minutes (0 - 4°C).

Le culot est remis en suspension dans un tampon Tris 50 mM pH 7,5 et le nouvel homogénat est centrifugé de nouveau à 30 000 g pendant 15 minutes (0 - 4°C).

Les culots, après élimination du surnageant, peuvent être utilisés aussitôt ou conservés jusqu'à 1 mois à -30°C.

Pour une expérience les culots sont d'abord décongelés, si nécessaire, à température ambiante et remis en suspension à l'aide d'un Dounce dans du tampon Tris 50 mM pH 7,5. Des aliquotes de 2 ml sont mis à incuber 60 minutes à 25°C en présence de 0,3 nM de ³H quinuclidinyl benzylate soit seul, soit avec le produit à tester, soit avec de la benzatropine à 10⁻⁵M pour déterminer la radioactivité fixée non spécifique.

A la fin de l'incubation, les tubes d'incubats sont refroidis à 4°C et filtrés rapidement sur filtres Whatman GF/C. Les filtres sont rincés par 3 x 5 ml de tampon Tris 50 mM pH 7,5 puis on effectue les mesures par scintillation liquide (Henry I Yamamura, Solomon H. Snyder, Proc. Nat. Acad. Sc. (1974) 71, n° 5, 1725-1729).

Les résultats sont exprimés en CI₅₀ (concentration nécessaire pour inhiber de 50% la radioactivité spécifique fixée).

**TABLEAU 1**

| Composé de l'exemple | Affinité pour les récepteurs muscariniques M₁ et M₂ | |
|---|---|---|
| | [³H]pirenzépine | [³H]quinuclidinyl benzylate |
| 2 | 314 | 4600 |
| 4 | 128 | 2240 |
| 5 | 49 | 660 |

Les composés des exemples 2, 4 et 5 montrent une remarquable et intéressante affinité pour le récepteur muscarinique, et principalement pour le récepteur de type M₁. Par contre les mêmes composés ont montré une affinité négligeable (CI₅₀ > 5000-10000) pour les autres récepteurs examinés parmi lesquels ont peut citer ceux de la dopamine, de l'histamine, de la sérotonine (5 HT₁ et 5 HT₂), des benzodiazépines, du GABA, des adrénorécepteurs (alpha1, alpha2, béta1, béta2) ou encore les récepteurs opiacés (mu, kappa).

### 2) Intéraction et affinité avec différents récepteurs intestinaux.

L'intéraction des composés avec différents récepteurs a été évaluée sur l'iléon isolé du cobaye selon la méthode suivante.

Des segments d'iléon de cobayes de 2,5 - 3 cm ont été lavés et immédiatement suspendus dans un bain contenant 10 ml d'une solution de Tyrode à 37°C et aérée avec un mélange d'oxygène (95%) et de gaz carbonique (5%). Après une période de stabilisation de 30 minutes au moins, on enregistre les contractions, en maintenant la préparation sous tension constante de 1 g, à l'aide d'un capteur relié à un polygraphe. On a évalué l'action agoniste en laissant le composé en contact avec le tissu isolé pendant une période nécessaire à exprimer la contraction maximale ; ensuite, on a lavé avec la solution de Tyrode. On n'a ajouté la dose suivante au bain qu'après que la préparation fût revenue sur sa ligne de base. Comme produit de référence, on a employé l'arécoline. On a évalué l'action antagoniste sur les contractions induites par l'acétylcholine (1 x 10⁻⁶M), l'histamine (1 x 10⁻⁵M) et le chlorure de baryum (2 x 10⁻⁴M). L'atropine, le diphénydramine et la papavérine ont été employés comme produits de référence. Le temps de contact avant d'ajouter l'agoniste a été d'une minute.

Pour chaque composé les courbes dose-réponse sont obtenues avec 4 à 6 concentrations différentes et 3 à 5 épreuves indépendantes. L'activité agoniste est exprimée par le pD₂ (logarithme négatif de la dose qui produit 50% de l'effet maximum). L'activité antagoniste est exprimée par la CI₅₀ (concentration inhibant 50% de la réponse maximale).

Les résultats obtenus avec les composés des exemples 2, 4 et 5 sont reportés dans le tableau suivant :

**TABLEAU 2**

| Composé de l'exemple | Antagoniste à différents agents (CI50:M) | | | Action agoniste pD₂ |
|---|---|---|---|---|
| | ACh | Histam. | BaCl₂ | |
| 2 | 9,3 x 10-7 | > 10-5 | > 10-5 | < 5 |
| 4 | 1,5 x 10-6 | > 10-5 | > 10-5 | < 5 |
| 5 | 2,7 x 10-7 | > 10-5 | > 10-5 | < 5 |
| Atropine | 9,5 x 10-9 | | | |
| Diphénydramine | | 8,3x10-7 | | |
| Papavérine | | | 4,5x10-5 | |
| Arécoline | | | | 6,68 |

Les études "in vitro" sur l'iléon isolé de cobaye ont mis en évidence que les composés de l'invention sont des agents antimuscariniques. Ils antagonisent les contractions induites par l'acétylcholine mais non celles induites par l'histamine et le chlorure de baryum.

### 3) Action anticholinergique "in vivo".

L'activité anticholinergique des composés a été déterminée en évaluant la capacité d'inhiber les effets cholinomimétiques induits par le carbachol. Le sulfate d'atropine a été employé comme produit de référence.

On utilise des souris mâles CD₁ pesant 25 à 30 g. Elles sont réparties en groupes de 6 animaux et traitées par voie intrapéritonéale à des doses scalaires des produits ou 0,25% de Méthocel pour les témoins. On utilise 12 animaux pour chaque dose. 30 minutes après l'administration des composés, on injecte aux souris par voie sous-cutanée 1 mg/kg de carbachol, dissous dans du sérum physiologique.

Chaque animal a été examiné 30 minutes après l'injection de carbachol pour évaluer la présence de diarrhée, salivation et larmoiement ; on a mesuré aussi la température corporelle au moyen d'un thermocouple inséré de 1,5 cm dans le rectum.

Le carbachol (1 mg/kg s.c.) a induit diarrhée, salivation et larmoiement chez toutes les souris témoins et une diminution de la température rectale de 2,5°C environ.

Pour chaque composé, nous avons déterminé et reporté dans le tableau suivant la dose capable d'inhiber chez 50% des animaux, l'apparition des symptômes cholinomimétiques induits par le carbachol et d'augmenter de 1°C l'effet hypothermique induit par l'agent cholinergique.

**TABLEAU 3**

| Composé de l'exemple | Dose mg/kg i.p. | | | Température corporelle |
|---|---|---|---|---|
| | Diarrhée | salivation | larmoiement | |
| 2 | 5 | > 50 | > 50 | > 50 |
| 4 | 6 | > 50 | > 50 | > 50 |
| 5 | 2 | ≃ 25 | > 50 | 50 |
| Atropine | 0,04 | 0,06 | 0,05 | 0,03 |

Les résultats obtenus montrent que, contrairement à l'atropine, les composés exercent "in vivo" une action anticholinergique sélective au niveau de la musculature intestinale.

## Revendications (Revendications pour l'(les) Etat(s) contractant(s) suivant(s): AT, BE, CH, DE, FR, GB, IT, LI, LU, NL, SE)

1. Les composés de formule (I) : 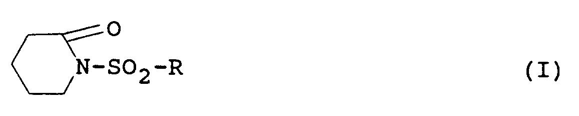 dans laquelle R représente un radical 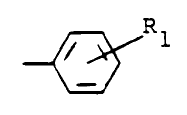 dans lequel R₁ en position quelconque sur le noyau phényle représente un radical 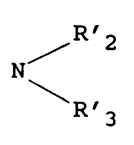 dans lequel R'₂ et R'₃ identiques ou différents représentent un radical alcoyle linéaire ou ramifié renfermant jusqu'à 8 atomes de carbone, ou forment avec l'atome d'azote auquel ils sont liés un radical hétérocyclique choisi parmi les radicaux pipéridyle, pipérazinyle, morpholinyle, pyrrolidinyle, hexahydroazépinyle et octahydroazocinyle.

2. Les composés de formule (I) tels que définis à la revendication 1, dans lesquels le radical R₁ est en position 4.

3. Les composés de formule (I) tels que définis à la revendication 1 ou 2, dans lesquels le radical R'₂ et R'₃ identiques ou différents, représentent un radical alcoyle renfermant jusqu'à 4 atomes de carbone.

4. Les composés de formule (I) tels que définis à la revendication 1 ou 2, dans lesquels R₁ représente un radical pipéridinyle.

5. Les composés de formule (I) tels que définis à la revendication 1 ou 2, dans lesquels R₁ représente un radical hexahydroazépinyle.

6. L'un quelconque des composés tels que définis à la revendication 1, dont les noms suivent :
- la 1-[4-(diéthylamino) benzènesulfonyl] 2-pipéridinone,
- la 1-[4-(1-pipéridinyl) benzènesulfonyl] 2-pipéridinone.

7. Procédé de préparation des composés de formule (I), tels que définis à la revendication 1, caractérisé en ce que l'on soumet un composé de formule (Il) :
R-SO₂-Hal (II)
dans laquelle Hal représente un atome de chlore ou de brome et R conserve la signification indiquée précédemment, à l'action de l'acide 5-amino valérique (III) : 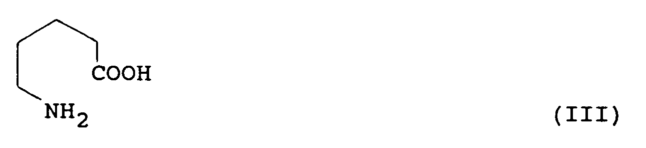 pour obtenir un composé de formule (IV) : 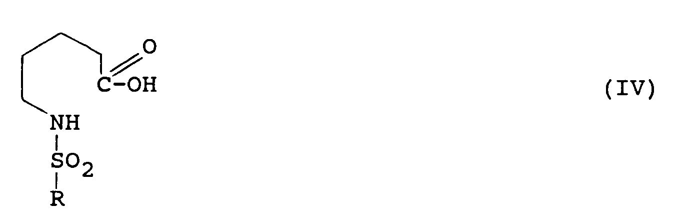 que l'on cyclise pour obtenir le produit de formule (I) correspondant.

8. A titre de médicaments, les composés de formule (I) tels que définis à l'une quelconque des revendications 1 à 5.

9. A titre de médicaments, le composé de formule (I) tel que défini à la revendication 6.

10. Les compositions pharmaceutiques renfermant au moins un médicament tel que défini à la revendication 8 ou 9.

## Revendications (Revendications pour l'(les) Etat(s) contractant(s) suivant(s): ES)

1. Procédé de préparation des composés de formule (I) : 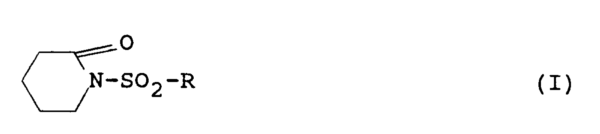 dans laquelle R représente un radical 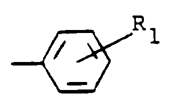 dans lequel R₁ en position quelconque sur le noyau phényle représente un radical 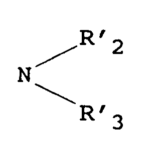 dans lequel R'₂ et R'₃ identiques ou différents représentent un radical alcoyle linéaire ou ramifié renfermant jusqu'à 8 atomes de carbone, ou forment ensemble avec l'atome d'azote auquel ils sont liés un radical hétérocyclique choisi parmi les radicaux pipéridyle, pipérazinyle, morpholinyle, pyrrolidinyle, hexahydroazépinyle et octahydroazocinyle, caractérisé en ce que l'on soumet un composé de formule (II) :
R-SO₂-Hal (II)
dans laquelle Hal représente un atome de chlore ou de brome et R conserve la signification indiquée précédemment, à l'action de l'acide 5-amino valérique (III) : 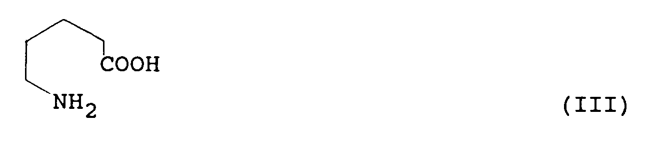 pour obtenir un composé de formule (IV) : 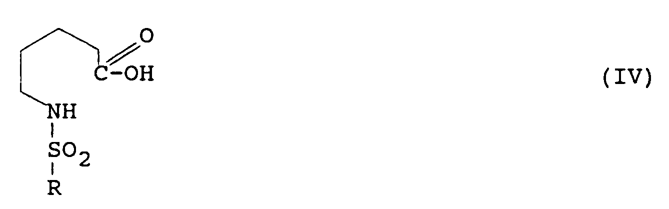 que l'on cyclise pour obtenir le produit de formule (I) correspondant.

2. Procédé selon la revendication 1, caractérisé en ce que le radical R₁ est en position 4.

3. Procédé selon la revendication 1 ou 2, caractérisé en ce que R'₂ et R'₃ identiques ou différents, représentent un radical alcoyle renfermant jusqu'à 4 atomes de carbone.

4. Procédé selon la revendication 1 ou 2, caractérisé en ce que R₁ représente un radical pipéridinyle.

5. Procédé selon la revendication 1, caractérisé en ce que l'on choisit le produit de formule (II) de manière telle que l'on prépare :
- la 1-[4-(diéthylamino) benzènesulfonyl] 2-pipéridinone,
- la 1-[4-(1-pipéridinyl) benzènesulfonyl] 2-pipéridinone.

## Revendications (Revendications pour l'(les) Etat(s) contractant(s) suivant(s): GR)

1. Procédé de préparation des composés de formule (I) : 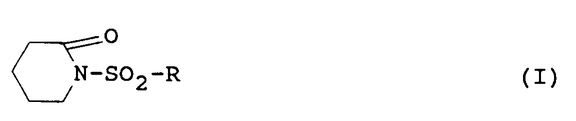 dans laquelle R représente un radical 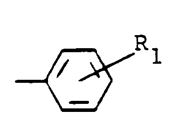 dans lequel R₁ en position quelconque sur le noyau phényle représente un radical 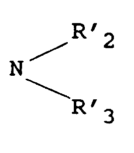 dans lequel R'₂ et R'₃ identiques ou différents représentent un radical alcoyle linéaire ou ramifié renfermant jusqu'à 8 atomes de carbone, ou forment ensemble avec l'atome d'azote auquel ils sont liés un radical hétérocyclique choisi parmi les radicaux pipéridyle, pipérazinyle, morpholinyle, pyrrolidinyle, hexahydroazépinyle et octahydroazocinyle,
caractérisé en ce que l'on soumet un composé de formule (II) :
R-SO₂-Hal (II)
dans laquelle Hal représente un atome de chlore ou de brome et R conserve la signification indiquée précédemment, à l'action de l'acide 5-amino valérique (III) : 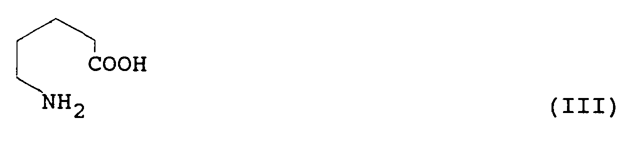 pour obtenir un composé de formule (IV) : 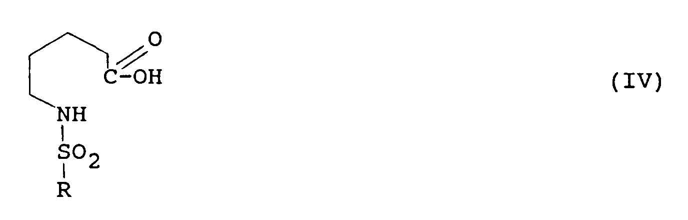 que l'on cyclise pour obtenir le produit de formule (I) correspondant.

2. Procédé selon la revendication 1, caractérisé en ce que le radical R₁ est en position 4.

3. Procédé selon la revendication 1 ou 2, caractérisé en ce que R'₂ et R'₃ identiques ou différents, représentent un radical alcoyle renfermant jusqu'à 4 atomes de carbone.

4. Procédé selon la revendication 1 ou 2, caractérisé en ce que R₁ représente un radical pipéridinyle.

5. Procédé selon la revendication 1, caractérisé en ce que l'on choisit le produit de formule (II) de manière telle que l'on prépare :
- la 1-[4-(diéthylamino) benzènesulfonyl] 2-pipéridinone,
- la 1-[4-(1-pipéridinyl) benzènesulfonyl] 2-pipéridinone.

6. Procédé de préparation des compositions pharmaceutiques caractérisé en ce que l'on met à titre de principe actif, l'un au moins des composés de formule (I) telle que définie à la revendication 1, sous une forme destinée à cet usage.

7. Procédé selon la revendication 6, caractérisé en ce que l'on utilise à titre de principe actif l'un au moins des dérivés de formule (I) tels qu'obtenus à la revendication 5.

## Claims (Claims for the following Contracting State(s): AT, BE, CH, DE, FR, GB IT, LI, LU, NL, SE)

1. The compounds of formula (I): 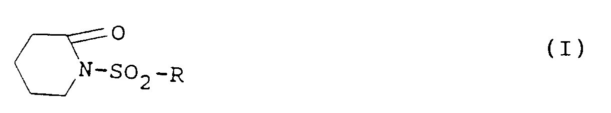 in which R represents a radical 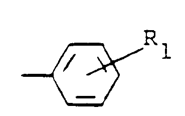 in which R₁ in any position on the phenyl nucleus represents a radical 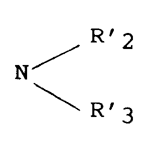 in which R'₂ and R'₃, identical or different, represent a linear or branched alkyl radical containing up to 8 carbon atoms, or form with the nitrogen atom to which they are linked a heterocyclic radical chosen from the following radicals: piperidyl, piperazinyl, morpholinyl, pyrrolidinyl, hexahydroazepinyl and octahydroazocinyl.

2. The compounds of formula (I) as defined in claim 1, in which the R₁ radical is in position 4.

3. The compounds of formula (I) as defined in claim 1 or 2, in which the R'₂ and R'₃ radical, identical or different, represent an alkyl radical containing up to 4 carbon atoms.

4. The compounds of formula (I) as defined in claim 1 or 2, in which R₁ represents a piperidinyl radical.

5. The compounds of formula (I) as defined in claim 1 or 2, in which R₁ represents a hexahydroazepinyl radical.

6. Any one of the compounds as defined in claim 1, of which the names follow:
- 1-[4-(diethylamino) benzenesulphonyl] 2-piperidinone,
- 1-[4-(1-piperidinyl) benzenesulphonyl] 2-piperidinone.

7. Preparation process for the compounds of formula (I), as defined in claim 1, characterized in that a compound of formula (II):
R-SO₂-Hal (II)
in which Hal represents a chlorine or bromine atom and R keeps the meaning indicated previously, is subjected to the action of 5-amino valeric acid (III): 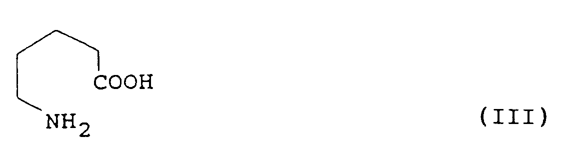 in order to obtain a compound of formula (IV): 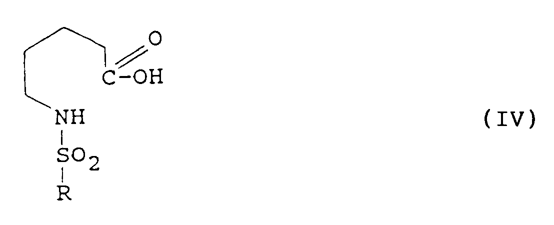 which is cyclized in order to obtain the corresponding product of formula (I).

8. As medicaments, the compounds of formula (I) as defined in any one of claims 1 to 5.

9. As medicaments, the compound of formula (I) as defined in claim 6.

10. Pharmaceutical compositions containing at least one medicament as defined in claim 8 or 9.

## Claims (Claims for the following Contracting State(s): ES)

1. Preparation process for compounds of formula (I): 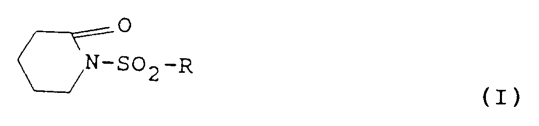 in which R represents a radical 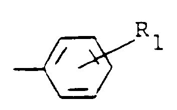 in which R₁ in any position on the phenyl nucleus represents a radical 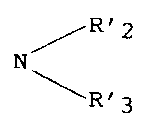 in which R'₂ and R'₃, identical or different, represent a linear or branched alkyl radical containing up to 8 carbon atoms, or form with the nitrogen atom to which they are linked a heterocyclic radical chosen from the following radicals: piperidyl, piperazinyl, morpholinyl, pyrrolidinyl, hexahydroazepinyl and octahydroazocinyl, characterized in that a compound of formula (II):
R-SO₂-Hal (II)
in which Hal represents a chlorine or bromine atom and R keeps the meaning indicated previously, is subjected to the action of 5-amino valeric acid (III): 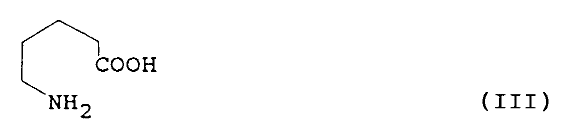 in order to obtain a compound of formula (IV): 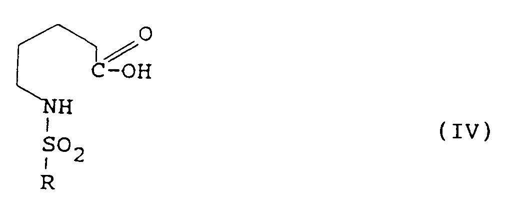 which is cyclized in order to obtain the corresponding product of formula (I).

2. Process according to claim 1, characterized in that the R₁ radical is in position 4.

3. Process according to claim 1 or 2, characterized in that R'₂ and R'₃, identical or different, represent an alkyl radical containing up to 4 carbon atoms.

4. Process according to claim 1 or 2, characterized in that R₁ represents a piperidinyl radical.

5. Process according to claim 1, characterized in that the product of formula (II) is chosen in such a way that the following are prepared:
- 1-[4-(diethylamino) benzenesulphonyl] 2-piperidinone,
- 1-[4-(1-piperidinyl) benzenesulphonyl] 2-piperidinone.

## Claims (Claims for the following Contracting State(s): GR)

1. Preparation process for compounds of formula (I): 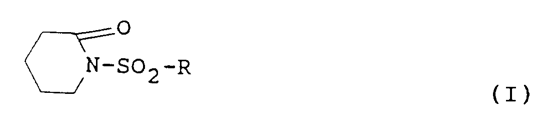 in which R represents a radical 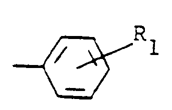 in which R₁ in any position on the phenyl nucleus represents a radical 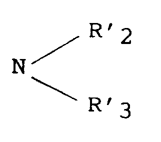 in which R'₂ and R'₃, identical or different, represent a linear or branched alkyl radical containing up to 8 carbon atoms, or form with the nitrogen atom to which they are linked a heterocyclic radical chosen from the following radicals: piperidyl, piperazinyl, morpholinyl, pyrrolidinyl, hexahydroazepinyl and octahydroazocinyl, characterized in that a compound of formula (II):
R-SO₂-Hal (II)
in which Hal represents a chlorine or bromine atom and R keeps the meaning indicated previously, is subjected to the action of 5-amino valeric acid (III): 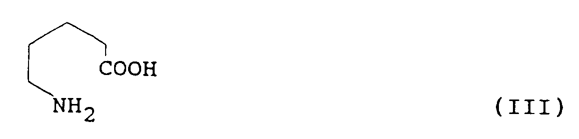 in order to obtain a compound of formula (IV): 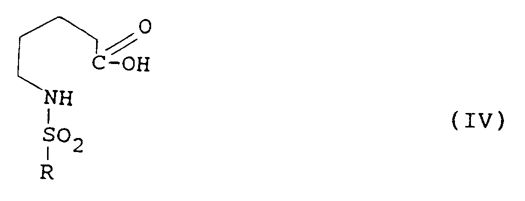 which is cyclized in order to obtain the corresponding product of formula (I).

2. Process according to claim 1, characterized in that the R₁ radical is in position 4.

3. Process according to claim 1 or 2, characterized in that R'₂ and R'₃, identical or different, represent an alkyl radical containing up to 4 carbon atoms.

4. Process according to claim 1 or 2, characterized in that R₁ represents a piperidinyl radical.

5. Process according to claim 1, characterized in that the product of formula (II) is chosen in such a way that the following are prepared:
- 1-[4-(diethylamino) benzenesulphonyl] 2-piperidinone,
- 1-[4-(1-piperidinyl) benzenesulphonyl] 2-piperidinone.

6. Preparation process for pharmaceutical compositions characterized in that at least one of the compounds of formula (I) as defined in claim 1 is used as active ingredient in a form intended for this use.

7. Process according to claim 6, characterized in that at least one of the derivatives of formula (I) as obtained in claim 5 is used as active ingredient.

## Patentansprüche (Patentansprüche für folgende(n) Vertragsstaat(en): AT, BE, CH, DE, FR, GB, IT, LI, LU, NL, SE)

1. Verbindungen der Formel (I): 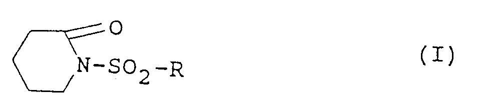 worin R für einen Rest 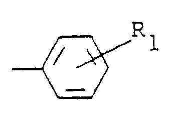 steht, worin R₁ an irgendeiner Position an dem Phenylring für einen Rest 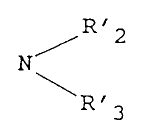 steht, worin R'₂ und R'₃, die gleich oder verschieden sind, für einen linearen oder verzweigten Alkylrest mit bis zu 8 Kohlenstoffatomen stehen, oder mit dem Stickstoffatom, an das sie gebunden sind, einen heterocyclischen Rest, ausgewählt aus Piperidyl-, Piperazinyl-, Morpholinyl-, Pyrrolidinyl-, Hexahydroazepinyl- und Octahydroazocinylresten, bilden.

2. Verbindungen der Formel (I) nach Anspruch 1, worin der Rest R₁ in Position 4 steht.

3. Verbindungen der Formel (I) nach Anspruch 1 oder 2, worin die Reste R'₂ und R'₃, die gleich oder verschieden sind, für einen Alkylrest mit bis zu 4 Kohlenstoffatomen stehen.

4. Verbindungen der Formel (I) nach Anspruch 1 oder 2, worin R₁ für einen Piperidinylrest steht.

5. Verbindungen der Formel (I) nach Anspruch 1 oder 2, worin R₁ für einen Hexahydroazepinylrest steht.

6. Eine der Verbindungen nach Anspruch 1, nämlich:
- 1-[4-(Diethylamino)benzolsulfonyl]-2-piperidinon,
- 1-[4-(1-Piperidinyl)benzolsulfonyl]-2-piperidinon.

7. Verfahren zur Herstellung der Verbindungen der Formel (I) nach Anspruch 1, dadurch **gekennzeichnet,** daß man eine Verbindung der Formel (II)
R-SO₂-Hal (II),
worin Hal für ein Chlor- oder Bromatom steht, und R wie vorstehend angegeben definiert ist, mit 5-Aminovaleriansäure (III) 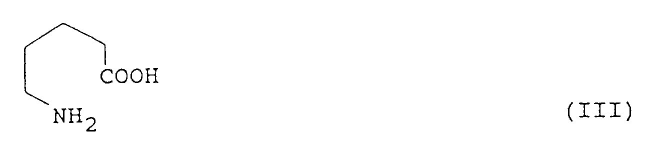 umsetzt, wodurch eine Verbindung der Formel (IV) 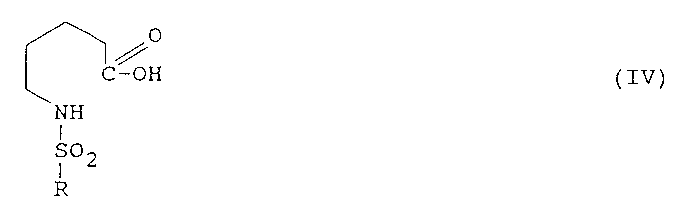 erhalten wird, die man cyclisiert, wodurch die entsprechende Verbindung der Formel (I) erhalten wird.

8. Verbindungen der Formel (I) nach einem der Ansprüche 1 bis 5 als Arzneimittel.

9. Verbindungen der Formel (I) nach Anspruch 6 als Arzneimittel.

10. Pharmazeutische Präparate, umfassend mindestens ein Arzneimittel nach Anspruch 8 oder 9.

## Patentansprüche (Patentansprüche für folgende(n) Vertragsstaat(en): ES)

1. Verfahren zur Herstellung der Verbindungen der Formel (I) 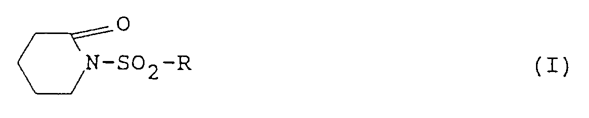 worin R für einen Rest 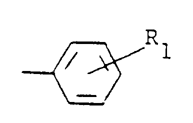 steht, worin R₁ an irgendeiner Position des Phenylrings für einen Rest 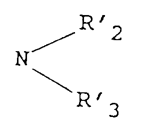 steht, worin R'₂ und R'₃, die gleich oder verschieden sind, für einen linearen oder verzweigten Alkylrest mit bis zu 8 Kohlenstoffatomen stehen, oder zusammen mit dem Stickstoffatom, an das sie gebunden sind, einen heterocyclischen Rest, ausgewählt aus Piperidyl-, Piperazinyl-, Morpholinyl-, Pyrrolidinyl-, Hexahydroazepinyl- und Octahydroazocinylresten, bilden, dadurch **gekennzeichnet,** daß man eine Verbindung der Formel (II)
R-SO₂-Hal (II),
worin Hal für ein Chlor- oder Bromatom steht, und R wie vorstehend angegeben definiert ist, mit 5-Aminovaleriansäure (III) 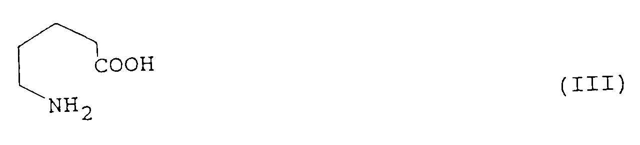 umsetzt, wodurch eine Verbindung der Formel (IV) 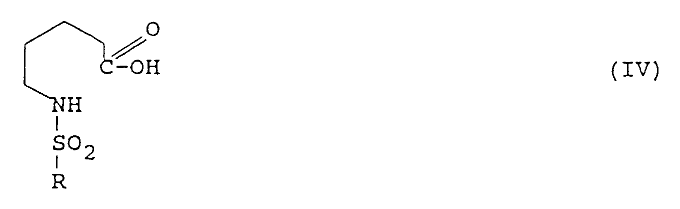 erhalten wird, die man cyclisiert, wodurch die entsprechende Verbindung der Formel (I) erhalten wird.

2. Verfahren nach Anspruch 1, dadurch **gekennzeichnet,** daß der Rest R₁ in Position 4 steht.

3. Verfahren nach Anspruch 1 oder 2, dadurch **gekennzeichnet,** daß R'₂ und R'₃, die gleich oder verschieden sind, für einen Alkylrest mit bis zu 4 Kohlenstoffatomen stehen.

4. Verfahren nach Anspruch 1 oder 2, dadurch **gekennzeichnet,** daß R₁ für einen Piperidinylrest steht.

5. Verfahren nach Anspruch 1, dadurch **gekennzeichnet,** daß man die Verbindung der Formel (II) so auswählt, daß man
- 1-[4-(Diethylamino)benzolsulfonyl]-2-piperidinon,
- 1-[4-(1-Piperidinyl)benzolsulfonyl]-2-piperidinon,
herstellt.

## Patentansprüche (Patentansprüche für folgende(n) Vertragsstaat(en): GR)

1. Verfahren zur Herstellung der Verbindungen der Formel (I) 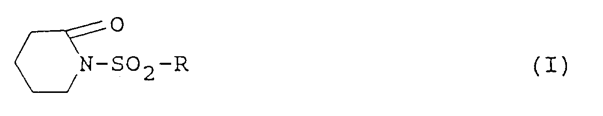 worin R für einen Rest 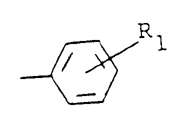 steht, worin R₁ an irgendeiner Position des Phenylrings für einen Rest 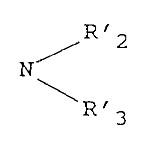 steht, worin R'₂ und R'₃, die gleich oder verschieden sind, für einen linearen oder verzweigten Alkylrest mit bis zu 8 Kohlenstoffatomen stehen, oder zusammen mit dem Stickstoffatom, an das sie gebunden sind, einen heterocyclischen Rest, ausgewählt aus Piperidyl-, Piperazinyl-, Morpholinyl-, Pyrrolidinyl-, Hexahydroazepinyl- und Octahydroazocinylresten, bilden, dadurch **gekennzeichnet,** daß man eine Verbindung der Formel (II)
R-SO₂-Hal (II),
worin Hal für ein Chlor- oder Bromatom steht, und R wie vorstehend angegeben definiert ist, mit 5-Aminovaleriansäure (III) 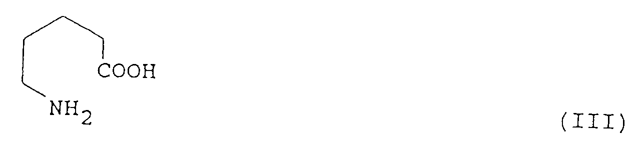 umsetzt, wodurch eine Verbindung der Formel (IV) 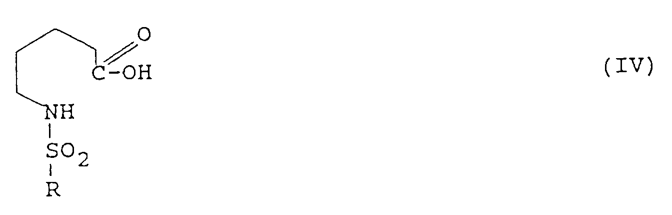 erhalten wird, die man cyclisiert, wodurch die entsprechende Verbindung der Formel (I) erhalten wird.

2. Verfahren nach Anspruch 1, dadurch **gekennzeichnet,** daß der Rest R₁ in Position 4 steht.

3. Verfahren nach Anspruch 1 oder 2, dadurch **gekennzeichnet,** daß R'₂ und R'₃, die gleich oder verschieden sind, für einen Alkylrest mit bis zu 4 Kohlenstoffatomen stehen.

4. Verfahren nach Anspruch 1 oder 2, dadurch **gekennzeichnet,** daß R₁ für einen Piperidinylrest steht.

5. Verfahren nach Anspruch 1, dadurch **gekennzeichnet,** daß man die Verbindung der Formel (II) so auswählt, daß man
- 1-[4-(Diethylamino)benzolsulfonyl]-2-piperidinon,
- 1-[4-(1-Piperidinyl)benzolsulfonyl]-2-piperidinon,
herstellt.

6. Verfahren zur Herstellung von pharmazeutischen Präparaten, dadurch **gekennzeichnet,** daß man als Wirkstoff mindestens eine der Verbindungen der Formel (I) nach Anspruch 1 in einer für diese Verwendung bestimmten Form einsetzt.

7. Verfahren nach Anspruch 6, dadurch **gekennzeichnet,** daß man als Wirkstoff mindestens eine der Verbindungen der Formel (I), erhalten gemäß Anspruch 5, verwendet.
